(19) Europäisches Patentamt
European Patent Office
Office européen des brevets

(11) Numéro de publication: 0 110 748
B1

# (12) FASCICULE DE BREVET EUROPEEN

(45) Date de publication du fascicule du brevet: **22.04.87**

(51) Int. Cl.[4]: **C 07 D 295/10**, A 61 K 31/55

(21) Numéro de dépôt: **83402070.3**

(22) Date de dépôt: **25.10.83**

(54) **Dérivés de phényl-(3-aminopropyl)-cétone, utilisation en thérapeutique et procédé de préparation.**

(30) Priorité: **26.10.82 FR 8217937**

(43) Date de publication de la demande: **13.06.84 Bulletin 84/24**

(45) Mention de la délivrance du brevet: **22.04.87 Bulletin 87/17**

(84) Etats contractants désignés: **AT BE CH DE FR GB IT LI LU NL SE**

(56) *Documents cités:*
**FR - A - 2 092 133**
**FR - A - 2 101 045**
**FR - A - 2 134 218**
**FR - M - 6 945**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Combe, André et al, CABINET BEAU DE LOMENIE 55 rue d'Amsterdam, F-75008 Paris (FR)**

EP 0 110 748 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne en tant que produits industriels de nouveaux dérivés de phényl-(3-aminopropyl)-cétone, à savoir la (2,4,6-triméthoxyphényl)-(3-hexaméthylèneiminopropyl)-cétone et ses sels d'addition. Elle concerne également l'utilisation en pharmacie et le procédé de préparation de ces nouveaux produits.

On sait que l'on a déjà décrit dans le passé un certain nombre de dérivés du type (alkoxy- et hydroxy-phényl)-(aminoalkyl)-cétone, voir à cet effet les brevets français No 1 492 256 et No 5636M et l'article de A. Boucherle et al., Chimie Thérapeutique 3 (No 4), 256–259, (1968), d'une part, les brevets britanniques No 1 078 975 et No 1 115 992, d'autre part, et enfin le brevet américain No 3 895 030, d'autre part.

On sait que les dérivés sus-visés du type (alkoxy- et hydroxy-phényl)-(aminoalkyl)-cétone ont été présentés en tant qu'agents anti-inflammatoires, antalgiques, antipyrétiques, antispasmodiques, tranquillisants, vasodilatateurs périphériques ou bradycardisants.

On sait enfin, notamment du brevet français No 2 404 003, qu'il n'y a pas de relation structure-activité à l'intérieur de la famille des (alkoxy- et hydroxy-phényl)-(aminoalkyl)-cétones, les effets pharmacologiques étant modifiés ou disparaissant en fonction de la nature des substituants du groupe phényle, de la nature du groupe amino et enfin de la nature du groupe alkyle présent entre le groupe CO et le groupe amino.

On vient de trouver de façon surprenante que la (2,4,6-triméthoxyphényl)-(3-hexaméthylène-iminopropyl)-cétone, qui est incluse dans la famille des (alkoxyphényl)-(3-aminopro pyl)-cétones envisagée dans la description du brevet américain No 3 895 030 précité sans être spécifiquement exemplifiée, présente, avec ses sels d'addition, des propriétés pharmacologiques intéressantes en thérapeutique et non évidentes compte tenu de l'enseignement de l'art antérieur.

En particulier, il n'était pas évident de l'enseignement du brevet américain No 3 895 030 qui décrit notamment (i) le chlorhydrate de (2,4,6-triméthoxyphényl)-(3-pyrrolidinopropyl)-cétone [produit commercialisé sous le nom de «FONZYLANE» (Dénomination Commune Internationale: CHLORHYDRATE de BUFLOMEDIL) et ayant pour No de code LL 1656] qui est un agent vasodilatateur périphérique de référence, et (ii) le chlorhydrate de (2,4,6-triméthoxyphényl)-(3-pipéridinopropyl)-cétone; [No de code LL 1647] qui est essentiellement un agent antispasmodique, que le chlorhydrate de (2,4,6-triméthoxyphényl)-(3-hexaméthylène-iminopropyl)-cétone [No de code CRL 41080] selon l'invention soit plus efficace que le LL 1656 en tant qu'agent vasodilatateur et bradycardisant, et dépourvu d'effet antispasmodique à la différence du LL 1647. En bref, il n'y a pas de relation structure-activité quand on passe du dérivé pyrrolidino au dérivé pipéridino puis au dérivé hexaméthylèneimino.

Les nouveaux dérivés de phényl-(3-aminopropyl)-cétone selon l'invention sont caractérisés en ce qu'ils sont choisis parmi l'ensemble constitué par

(i) la (2,4,6-triméthoxyphényl)-(3-hexaméthylèneiminopropyl)-cétone qui a pour formule développée

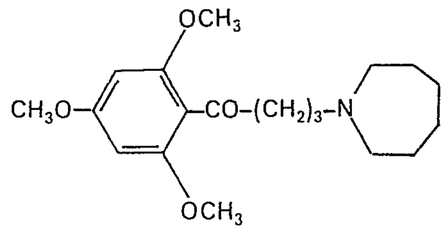

(I)

(ii) ses sels d'addition.

Par sels d'addition, on entend ici, d'une part, les sels d'addition d'acide obtenus par réaction de la base libre de formule I avec les acides minéraux et organiques, et, d'autre part, les sels d'ammonium. Parmi les acides utilisables pour salifier la base de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. Les sels d'addition d'acide sont les sels préférés, le chlorhydrate de (2,4,6-triméthoxyphényl)-(3-hexaméthylèneiminopropyl)-cétone étant particulièrement intéressant sur le plan thérapeutique.

La base de formule I peut être préparée selon une méthode connue en soi par application de mécanismes réactionnels classiques. Le procédé que l'on préconise selon l'invention comprend:

a) la réaction de l'hexaméthylèneimine avec le 4-chlorobutyronitrile, puis

b) la réaction du 4-hexaméthylèneimino-butyronitrile ainsi obtenu avec le 1,3,5-triméthoxybenzène en présence d'un courant gazeux de HCl à une température comprise entre -5°C et 0°C pendant au moins 2 h dans un solvant anhydre (notamment choisi parmi l'ensemble constitué par le benzène, le toluène, le chlorobenzène et leurs mélanges), puis la soumission du dérivé cétimine ainsi obtenu à une réaction d'hydrolyse pendant au moins 0,5 h à la température de reflux du milieu réactionnel.

De façon avantageuse, la réaction du stade a) sera réalisée à partir de 2 moles d'hexaméthylèneimine pour 1 mole de 4-chlorobutyronitrile en présence d'un hydrocarbure aromatique tel que le benzène ou le toluène, pendant au moins 4 h à la température de reflux du milieu réactionnel; et la réaction du stade b) entre le 4-hexaméthylèneimino-butyronitrile et le 1,3,5-triméthoxybenzène sera réalisée dans des conditions stoechiométriques au sein du chlorobenzène, le dérivé cétimine formé étant ensuite hydrolysé sans avoir été isolé.

La (2,4,6-triméthoxyphényl)-(3-hexaméthylèneiminopropyl)-cétone et ses sels sont des

agents utiles dans le traitement des maladies du système cardiovasculaire; ils agissent en tant qu'agents vasodilatateurs et bradycardisants par voie intraveineuse et intraduodénale. Selon l'invention, on préconise une composition thérapeutique qui est caractérisée en ce qu'elle renferme, en association avec un excipient physiologiquement acceptable, au moins un composé choisi parmi l'ensemble constitué par la (2,4,6-triméthoxyphényl)-(3-hexaméthylèneiminopropyl) -cétone et ses sels d'addition non toxiques. Dans une telle composition, l'ingrédient actif intervient bien entendu à une dose pharmaceutiquement efficace.

D'autres avantages et caractéristiques de l'invention seront mieux compris à la lecture qui va suivre (i) d'un exemple de préparation nullement limitatif mais donné à titre d'illustration, et (ii) des résultats des essais pharmacologiques qui ont été mis en œuvre.

Préparation

Obtention du chlorhydrate de (2,4,6-triméthoxyphényl) -(3-hexaméthylèneiminopropyl)-cétone (No de code CRL 41 080)

a) 4-Hexaméthylèneimino-butyronitrile

Au sein d'une solution au reflux de 50 g (0,505 mole) d'hexaméthylèneimine dans 80 ml de benzène, on coule en 15 min 26,1 g (0,252 mole) de 4-chlorobutyronitrile. On poursuit le reflux 4 h, on reprend le milieu réactionnel froid par de l'éther diéthylique, on élimine le précipité par filtration et on amène le filtrat à siccité. Le résidu ainsi obtenu est purifié par une distillation sous pression réduite pour donner 29,3 g (rendement 70%) de 4-hexaméthylèneimino-butyronitrile qui se présente sous la forme d'une huile incolore.

$Eb_{6\text{-}7\ mm\ Hg}$ = 126°C (6-7 mm Hg correspondent approximativement à 800-993 pascals).

b) CRL 41 080

On fait passer entre -5°C et 0°C pendant 2,5 h un courant d'acide chlorhydrique sec au sein d'une solution de 16,8 g (0,10 mole) de 1,3,5-triméthoxybenzène et de 16,6 g (0,10 mole) de 4-hexaméthylèneimino-butyronitrile en solution dans 115 ml de chlorobenzène anhydre. On abandonne la suspension au froid entre 0°C et +4°C une nuit et on extrait le milieu réactionnel par de l'eau. On porte la phase aqueuse au reflux pendant 1 h, on alcalinise par de la soude concentrée et on extrait le précipité par de l'éther diéthylique pour donner 29,8 g d'une huile jaune pâle. Cette huile est traitée dans l'éther diéthylique par de l'éthanol chlorhydrique. Après purification du précipité par cristallisation dans le mélange acétate d'éthyle-isopropanol (8:7) v/v, on obtient 19,5 g (rendement du stade b): 52,5%; rendement global: 38,75%) de CRL 41 080 se présentant sous la forme d'une poudre blanche soluble dans l'eau. $F_{inst}$ (Kofler) = 150°C.

On a résumé ci-après les résultats des essais pharmacologiques qui ont été entrepris avec le CRL 41 080.

I – Propriétés vasodilatatrices du CRL 41080

A – Action vasodilatatrice femorale par voie intraveineuse

Deux chiens (poids moyen 12,4 kg) anesthésiés au pentobarbital reçoivent le CRL 41 080 par voie intraveineuse, en perfusion de 6 min, aux doses successives de 2 fois 1 mg/kg, puis 2 et 4 mg/kg toutes les 60 min. Pour comparaison, ces animaux reçoivent en fin d'essai une perfusion de 6 mg/kg I.V. d'un produit de référence connu, le LL 1656 précité.

On constate que le CRL 41 080 ne modifie pas la pression artérielle ni la fréquence cardiaque; le débit fémoral augmente pendant la durée de la perfusion dès 1 mg/kg, l'effet est reproductible et s'accroît modérément avec la dose. Le LL 1656, à la dose de 6 mg/kg, augmente moins le débit fémoral que 1 mg/kg de CRL 41 080.

B – Action vasodilatatrice par voie intraduodenale

Quatre chiens (poids moyen 15,7 kg) anesthésiés au nembutal reçoivent le CRL 41 080 par voie intraduodénale, aux doses successives de 0,5 mg/kg, 1 mg/kg, 2,5 mg/kg et 5 mg/kg; trois de ces chiens reçoivent ensuite 10 mg/kg de CRL 41 080. On mesure la pression artérielle, la fréquence cardiaque, le débit artériel fémoral, le débit artériel vertébral, les températures rectale et cutanée.

On constate que le CRL 41 080 augmente nettement le débit fémoral à partir de la dose de 5 mg/kg; cet effet dure plus d'une heure. Par ailleurs, il semble que le débit vertébral soit diminué, mais cet effet n'est pas statistiquement significatif.

C – Duree de l'action vasodilatatrice

Trois chiens (poids moyen 11,1 kg) reçoivent le CRL 41 080 par voie intraduodénale à la dose de 5 mg/kg à deux reprises (délai entre les deux administrations: 90 min) puis 6 mg/kg I.D. de LL 1656 et, 2 heures plus tard, une nouvelle administration de 5 mg/kg I.D. de CRL 41 080.

On observe que le CRL 41 080 augmente le débit fémoral à 5 mg/kg I.D.; cet effet est maximal à 30 min, a perdu la moitié de son intensité à 60 min et a disparu à 120 min; les autres paramètres mesurés ne sont pas modifiés. Une deuxième administration de 5 mg/kg de CRL 41 080 a un effet plus intense sur le débit fémoral, l'effet est maximal à 30 min, diminue ensuite progressivement, mais est encore présent à 120 min. Une administration de 6 mg/kg I.D. de LL 1656 entraîne une augmentation de débit fémoral d'intensité moyenne, lente à se développer puis le maximum de l'effet est obtenu à 120 min. Cette cinétique d'action du LL 1656 par voie intraduodénale n'est pas la cinétique habituelle (maximum de l'effet à 30 ou 60 min et disparition progressive de celui-ci ensuite); on peut penser que l'administration préalable de CRL 41 080 modifie l'action du LL 1656. Une dose supplémentaire de 5 mg/kg I.D. de 41 080 n'augmente pas davantage le débit fémoral mais entraîne une hypotension et une bradycardie. Chez ces chiens, le CRL 41 080 ne diminue pas le débit vertébral.

D - Conclusion

Les résultats des essais résumés ci-dessus mettent en évidence que le CRL 41 080 est un agent vasodilatateur périphérique (notamment un vasodilatateur fémoral) et bradycardisant particulièrement intéressant. Le CRL 41 080 est plus actif en tant que vasodilatateur que le LL 1656, son homologue pyrrolidino, et ne présente pas les propriétés antispasmodiques de son homologue pipéridino.

II - Essais comparatifs

On a consigné dans les tableaux I et II ci-après les résultats des essais complémentaires qui ont été entrepris pour comparer le CRL 41 080 selon l'invention avec ses homologues pyrrolidino (LL 1656) et pipéridino (LL 1647) décrits dans le brevet américain No 3 895 030 précité.

Le tableau I concerne la toxicité chez la souris mâle, d'une part, et la dose minimale induisant chez le chien anesthésié une action vasodilatatrice fémorale nette, d'autre part. La DL-50 I.V. a été déterminée sur des lots de 10 souris mâles; la dose minimale vasodilatatrice a été déterminée sur un lot de 5 chiens servant de témoins par rapport à eux-mêmes et recevant chaque dose de produit à tester toutes les 2,5 heures.

Le tableau II concerne les variations des pressions artérielles (P.A. systolique, diastolique, différentielle et moyenne, de la fréquence cardiaque et du débit artériel fémoral. Ces variations ont été déterminées par voie intraveineuse sur le même lot précité de 5 chiens anesthésiés.

Au cours des essais cliniques, on a obtenu de bons résultats chez l'homme en administrant le CRL 41 080 par voie orale (trois gélules par jour renfermant chacune 100 mg d'ingrédient actif) et par voie injectable (voie I.V. ou voie I.M., une ampoule de 5 ml par jour renfermant 40 mg de principe actif dans un soluté physiologique.

Tableau I

| Produit | DL-50 I.V. souris | dose minimale induisant chez le chien anesthésié une action vasodilatatrice fémorale | |
|---|---|---|---|
| | | par voie I.V. | par voie I.D. |
| CRL 41080 (a) | 96±3 mg/kg | 1 mg/kg | 5 mg/kg |
| LL 1656 (b) (c) | 60±2,2 mg/kg | 5 mg/kg | 6 mg/kg |
| LL 1647 (c) | 68±4 mg/kg | 5 mg/kg | (d) |

Notes

(a) produit selon l'invention

(b) vasodilatateur périphérique de référence

(c) décrit dans brevet américain No 3 895 030

(d) pas d'action vasodilatatrice par voie I.D.

Tableau II

Propriétés cardiovasculaires chez le chien anesthésié

| Produit | Dose (mg/kg I.V.) | Variation 0,25 h après administration | | | | | |
|---|---|---|---|---|---|---|---|
| | | P.A. systolique | P.A. diastolique | P.A. différentielle | P.A. moyenne | Fréquence cardiaque | Débit fémorale |
| LL 1656 | 6 | −10 | −12 | +1 | −12 | 0 | +27 (a) |
| | 1 | 0 | −3 | −9 | −4 | +1 | +20 (b) |
| CRL 41080 | 2 | +2 | −1 | +15 | 0 | +3 | +39 |
| | 4 | −2 | −1 | −5 | −1 | −6 | +82 |

Notes

(a): +32% à la 6è minute; (b): +51% à la 6è minute.

**Revendications pour les Etats contractants BE, CH, DE, FR, GB, IT, LI, LU, NL, SE,**

1. La (2,4,6-triméthoxyphényl)-(3-hexaméthylèneiminopropyl)-cétone qui a pour formule développée

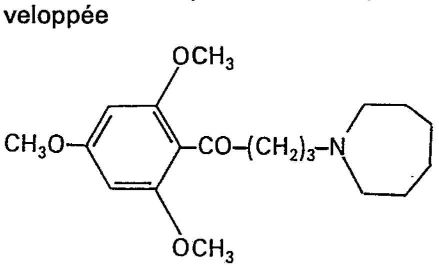

(I)

et ses sels d'addition.

2. Chlorhydrate de (2,4,6-triméthoxyphényl)-(3-hexaméthylèneiminopropyl)-cétone.

3. Composition thérapeutique, caractérisée en ce qu'elle renferme, en association avec un excipient physiologiquement acceptable, au moins un principe actif choisi parmi l'ensemble constitué par la (2,4,6-triméthoxyphényl)-(3-hexaméthylèneiminopropyl)-cétone et ses sels d'addition.

4. Composition selon la revendication 3, caractérisée en ce que le principe actif qu'elle renferme est le chlorhydrate de (2,4,6-triméthoxyphényl)-(3-hexaméthylèneiminopropyl)-cétone.

5. Procédé de préparation de la (2,4,6-triméthoxyphényl)-(3-hexaméthylèneiminopropyl) -cétone, dans lequel on forme le 4-hexaméthylèneimino-butyronitrile, son dérivé cétimine par réaction avec le 1,3,5-triméthoxybenzène, et hydrolyse ledit dérivé cétimine, ledit procédé étant caractérisé en ce que

a) on fait réagir 2 moles d'hexaméthylèneimine avec 1 mole de 4-chlorobutyronitrile, en présence d'un hydrocarbure aromatique pendant au moins 4 h à la température de reflux du milieu réactionnel, puis

b) on fait réagir 1 mole du 4-hexaméthylèneimino-butyronitrile ainsi formé, avec 1 mole de 1,3,5-triméthoxybenzène, en présence d'un courant gazeux de HCl, à une température comprise entre -5°C et 0°C pendant au moins 2 h dans un solvant anhydre, puis soumet le dérivé cétimine correspondant ainsi obtenu à une réaction d'hydrolyse pendant au moins 0,5 h à la température de reflux du milieu réactionnel.

6. Procédé selon la revendication 5, caractérisé en ce que dans l'étape a) l'hydrocarbure aromatique est le benzène ou le toluène et en ce que dans l'étape b) le solvant anhydre est choisi parmi le benzène, le toluène, le chlorobenzène ou leurs mélanges.

**Revendications pour l'Etat contractant AT**

1. Utilisation de la (2,4,6-triméthoxyphényl)-(3-hexaméthylèneiminopropyl)-cétone qui a pour formule développée

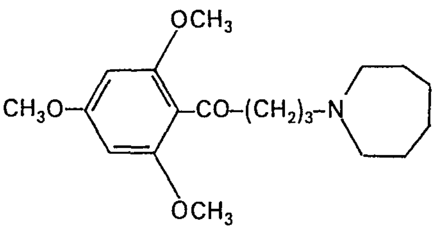

(I)

et ses sels d'addition non toxiques pour la fabrication d'un médicament.

2. Utilisation selon la revendication 1, caractérisée en ce que le sel est le chlorhydrate de (2,4,6-triméthoxyphényl)-(3-hexaméthylèneiminopropyl)-cétone.

3. Utilisation selon l'une des revendications 1 ou 2, caractérisée en ce que au moins un principe actif choisi parmi l'ensemble constitué par la (2,4,6-triméthoxyphényl)-(3-hexaméthylèneiminopropyl)-cétone et se sels d'addition non toxiques est en association avec un excipient physiologiquement acceptable.

4. Utilisation selon la revendication 3, caractérisée en ce que le principe actif est le chlorhydrate de (2,4,6-triméthoxyphényl)-(3-hexaméthylèneiminopropyl)-cétone.

5. Procédé de préparation de la (2,4,6-triméthoxyphényl)-(3-hexaméthylèneiminopropyl)-cétone, dans lequel on forme le 4-hexaméthylèneimino-butyronitrile, son dérivé cétimine par réaction avec le 1,3,5-triméthoxybenzène, et hydrolyse ledit dérivé cétimine, ledit procédé étant caractérisé en ce que

a) on fait réagir 2 moles d'hexaméthylèneimine avec 1 mole de 4-chlorobutyronitrile, en présence d'un hydrocarbure aromatique pendant au moins 4 h à la température de reflux du milieu réactionnel, puis

b) on fait réagir 1 mole du 4-hexaméthylèneimino-butyronitrile ainsi formé, avec 1 mole de 1,3,5-triméthoxybenzène, en présence d'un courant gazeux de HCl, à une température comprise entre -5°C et 0°C pendant au moins 2 h dans un solvant anhydre puis soumet le dérivé cétimine correspondant ainsi obtenu à une réaction d'hydrolyse pendant au moins 0,5 h à la température de reflux du milieu réactionnel.

6. Procédé selon la revendication 5, caractérisé en ce que dans l'étape a) l'hydrocarbure aromatique est le benzène ou le toluène et en ce que dans l'étape b) le solvant anhydre est choisi parmi le benzène, le toluène, le chlorobenzène ou leurs mélanges.

**Patentansprüche für die Vertragsstaaten BE, CH, DE, FR, GB, IT, LI, LU, NL, SE:**

1. (2,4,6-Trimethoxyphenyl)-(3-hexamethyleniminopropyl)-keton der Strukturformel

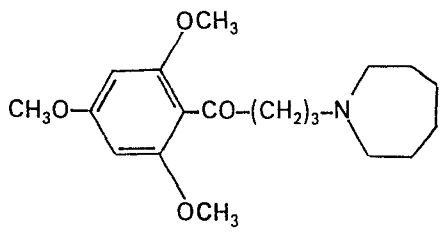

(I)

und seine Additionssalze.

2. (2,4,6-Trimethoxyphenyl)-(3-hexamethyleniminopropyl)-keton-hydrochlorid.

3. Therapeutische Zusammensetzung, dadurch gekennzeichnet, dass sie in Kombination mit einem physiologisch akzeptablen Exzipienten mindestens einen Wirkstoff, ausgewählt aus der Gruppe bestehend aus (2,4,6-Trimethoxyphenyl)-(3-hexamethyleniminopropyl)-keton und seinen Additionssalzen, umfasst.

4. Zusammensetzung nach Anspruch 3, dadurch gekennzeichnet, dass der Wirkstoff, den sie enthält, (2,4,6-Trimethoxyphenyl)-(3-hexamethyleniminopropyl)-keton-hydrochlorid ist.

5. Verfahren zur Herstellung von (2,4,6-Trimethoxyphenyl)-(3-hexamethyleniminopropyl)-keton, bei welchem man 4-Hexamethylenimino-butyronitril, sein Ketiminderivat, durch Umsetzung mit 1,3,5-Trimethoxybenzol bildet und das Ketiminderivat hydrolysiert, welches Verfahren dadurch gekennzeichnet ist, dass man

a) 2 Mol Hexamethylenimin mit 1 Mol 4-Chlorbutyronitril in Gegenwart eines aromatischen Kohlenwasserstoffs während mindestens 4 h bei Rückflusstemperatur des Reaktionsmilieus reagieren lässt, und

b) 1 Mol des so gebildeten 4-Hexamethylenimino-butyronitrils mit 1 Mol 1,3,5-Trimethoxybenzol in Gegenwart eines HCl-Gasstroms bei einer Temperatur zwischen -5°C und 0°C während mindestens 2 h in einem wasserfreien Lösungsmittel reagieren lässt und dann das so erhaltene entsprechende Ketiminderivat während mindestens 0,5 h bei Rückflusstemperatur des Reaktionsmilieus einer Hydrolysereaktion unterzieht.

6. Verfahren nach Anspruch 5, dadurch gekennzeichnet, dass der aromatische Kohlenwasserstoff in Stufe a) Benzol oder Toluol ist und dass das wasserfreie Lösungsmittel in Stufe b) ausgewählt ist aus Benzol, Toluol, Chlorbenzol oder Mischungen davon.

**Patentansprüche für den Vertragsstaat AT**

1. Verwendung von (2,4,6-Trimethoxyphenyl)-(3-hexamethyleniminopropyl)-keton der Strukturformel

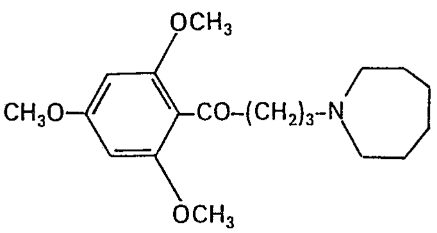 (I)

und seiner nichttoxischen Additionssalze, zur Herstellung eines Medikaments.

2. Verwendung nach Anspruch 1, dadurch gekennzeichnet, dass das Salz (2,4,6-Trimethoxyphenyl)-(3-hexamethyleniminopropyl)-keton-hydrochlorid ist.

3. Verwendung nach einem der Ansprüche 1 oder 2, dadurch gekennzeichnet, dass mindestens ein Wirkstoff, ausgewählt aus der Gruppe bestehend aus (2,4,6-Trimethoxyphenyl)-(3-hexamethyleniminopropyl)-keton und seinen nichttoxischen Additionssalzen, mit einem physiologisch akzeptablen Exzipienten kombiniert ist.

4. Verwendung nach Anspruch 3, dadurch gekennzeichnet, dass der Wirkstoff (2,4,6-Trimethoxyphenyl)-(3-hexamethyleniminopropyl)-keton-hydrochlorid ist.

5. Verfahren zur Herstellung von (2,4,6-Trimethoxyphenyl)-(3-hexamethyleniminopropyl)-keton, bei welchem man 4-Hexamethylenimino-butyronitril, sein Ketiminderivat, durch Umsetzung mit 1,3,5-Trimethoxybenzol bildet und das Ketiminderivat hydrolysiert, welches Verfahren dadurch gekennzeichnet ist, dass man

a) 2 Mol Hexamethylenimin mit 1 Mol 4-Chlorbutyronitril in Gegenwart eines aromatischen Kohlenwasserstoffs während mindestens 4 h bei Rückflusstemperatur des Reaktionsmilieus reagieren lässt, und b) 1 Mol des so gebildeten 4-Hexamethylenimino-butyronitrils mit 1 Mol 1,3,5-Trimethoxybenzol in Gegenwart eines HCl-Gasstroms bei einer Temperatur zwischen -5°C und 0°C während mindestens 2 h in einem wasserfreien Lösungsmittel reagieren lässt und dann das so erhaltene entsprechende Ketiminderivat während mindestens 0,5 h bei Rückflusstemperatur des Reaktionsmilieus einer Hydrolysereaktion unterzieht.

6. Verfahren nach Anspruch 5, dadurch gekennzeichnet, dass der aromatische Kohlenwasserstoff in Stufe a) Benzol oder Toluol ist, und dass das wasserfreie Lösungsmittel in Stufe b) ausgewählt ist aus Benzol, Toluol, Chlorbenzol oder Mischungen davon.

**Claims for the contracting state AT**

1. Use of the (2,4,6-trimethoxyphenyl)-3-(hexamethyleneiminopropyl)-ketone having the following structural formula

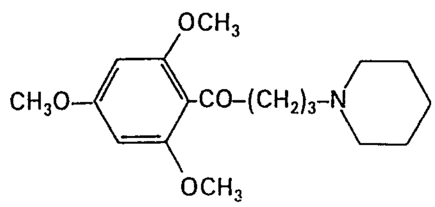 (I)

and its non toxic addition salts, for the manufacture of a medicine.

2. Use according to claim 1, characterized in that the salt is the (2,4,6-trimethoxyphenyl)-(3-hexamethyleneiminopropyl)-ketone hydrochlorate.

3. Use according to any one of claims 1 or 2, characterized in that at least one active principle selected from the group constituted by the (2,4,6-trimethoxyphenyl)-(3-hexamethyleneiminopropyl)-ketone and its non toxic addition salts is in association with a physiologically acceptable excipient.

4. Use according to claim 3, characterized in that the active principle is the (2,4,6-trimethoxyphenyl)- (3-hexamethyleneiminopropyl)-ketone-hydrochlorate.

5. Process for preparing the (2,4,6-trimethoxyphenyl) -(3-hexamethyleneiminopropyl)-ketone, in which the 4-hexamethyleneimino-butyronitrile is formed, its ketimine derivative being formed by reaction with the 1,3,5-trimethoxybenzene, said ketimine derivative being hydrolyzed, said process being characterized in that

a) 2 mols of hexamethyleneimine are reacted with 1 mol of 4-chlorobutyronitrile, in the presence of an aromatic hydrocarbon, for at least 4 hours at the reflux temperature of the reaction medium, then

b) 1 mol of 4-hexamethyleneimino-butyronitrile thus obtained is reacted with 1 mol of 1,3,5-trimethoxybenzene, in the presence of a gaseous stream of HCl, at a temperature of between -5°C and 0°C for at least 2 hours in an anhydrous solvent, then the corresponding ketimine derivative thus obtained is subjected to a hydrolysis reaction for at least 0.5 hour at the reflux temperature of the reaction medium.

6. Process according to claim 5, characterized in that in step a) the aromatic hydrocarbon is benzene or toluene and in that in step b) the anhydrous solvent is selected from among benzene, toluene, chlorobenzene or the mixtures thereof.

**Claims for the contracting states BE, CH, DE, FR, GB, IT, LI, LU, NL, SE.**

1. The (2,4,6-trimethoxyphenyl)-(3-hexamethyleneiminopropyl)-ketone having the following structural formula

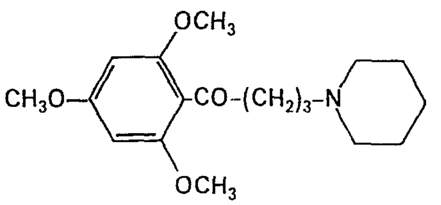

(I)

and its addition salts.

2. (2,4,6-trimethoxyphenyl)-(3-hexamethyleneiminopropyl)-ketone hydrochlorate.

3. Therapeutical composition, characterized in that it comprises in association with a physiologically acceptable excipient, at least one active principle selected from the group constituted by the (2,4,6-trimethoxyphenyl)-(3-hexamethyleneiminopropyl)-ketone and its addition salts.

4. Composition according to claim 3, characterized in that the active principle which it contains is the (2,4,6-trimethoxyphenyl)-(3-hexamethyleneiminopropyl)-ketone hydrochlorate.

5. Process for preparing the (2,4,6-trimethoxyphenyl)-(3-hexamethyleneiminopropyl)-ketone, in which the 4-hexamethyleneimino-butyronitrile is formed, its ketimine derivative being formed by reaction with the 1,3,5-trimethoxybenzene, said ketimine derivative being hydrolyzed, said process being characterized in that

a) 2 mols of hexamethyleneimine are reacted with 1 mol of 4-chlorobutyronitrile, in the presence of an aromatic hydrocarbon, for at least 4 hours at the reflux temperature of the reaction medium, then

b) 1 mol of 4-hexamethyleneimino-butyronitrile thus formed with 1 mol of 1,3,5-trimethoxybenzene, in the presence of a gaseous stream of HCl, at a temperature of between -5°C and 0°C for at least 2 hours in an anhydrous solvent, then the corresponding ketimine derivative thus obtained is subjected to a hydrolysis reaction for at least 0.5 hour at the reflux temperature of the reaction medium.

6. Process according to claim 5, characterized in that in step a) the aromatic hydrocarbon is benzene or toluene and in that in step b) the anhydrous solvent is selected from among benzene, toluene, chlorobenzene or the mixtures thereof.